# DEMANDE DE BREVET EUROPEEN

(11) **EP 1 757 266 A1**
(43) Date de publication de la demande: **28.02.2007**
(21) Numéro de dépôt: 06118518.7
(22) Date de dépôt: 07.08.2006
(51) Int. Cl.: A61K 8/81, A61K 8/31, A61K 8/87, A61Q 1/10, A61Q 1/12

(54) **Fard à paupières sous forme de poudre compact comprenant du polydécène liquide**

(30) Priorité: 26.08.2005 FR 0552572
(71) Demandeur: L'Oreal, 75008 Paris (FR)
(72) Inventeur: Weinling, Aurélie, 75015, Paris (FR); Champenois, Sandrine, 92230, Gennevilliers (FR)
(74) Mandataire: Duvert, Sandra

(57) **Abrégé**

L'invention a pour objet un fard à paupières comprenant, dans un milieu cosmétiquement acceptable, au moins un agent de coloration choisi parmi les pigments diffractants, les pigments interférentiels, les pigments réféchissants et leurs mélanges, une phase grasse comprenant du polydécène liquide à 25 °C , au moins une cire présente totalement ou partiellement sous forme de poudre et/ou une poudre de polyuréthane, ledit présentant une brillance supérieure ou égale à 1000 coûts.

## Description

La présente invention a pour objet une composition cosmétique de fard-à-paupières comprenant un phase grasse liquide particulière, ladite composition étant caractérisée par sa brillance. L'invention a aussi pour objet un procédé de maquillage des paupières utilisant la composition.

Les fards à paupières sont constituées d'un véhicule approprié et de différentes matières colorantes destinées à conférer un certain effet coloriel au fard et après leur application sur les paupières. Ils peuvent se présenter sous forme aqueuse ou anhydre.

De nombreuses compositions de maquillage de ce type se présentent sous forme de poudre compacte comprenant généralement une phase grasse, appelée classiquement « liant », et une phase pulvérulente comprenant notamment des agents de coloration (pigments, nacres) et/ou des charges. Le liant a pour fonction principale de garantir une cohésion suffisante de la composition finale, notamment de manière a lui éviter une fragmentation pouvant être provoquée par les chocs et de lui conférer par ailleurs une bonne aptitude au prélèvement.
En fait, l'obtention de ces propriétés implique par ailleurs que les autres composants de la composition cosmétique, et en particulier les charges qu'elle contient s'avèrent également appropriées pour une mise en forme de type poudre compacte.

On cherche à obtenir des fards à paupières présentant un effet coloriel intense et ce en particulier par l'incorporation d'un taux élevé de pigments, en particulier des nacres.

Or, ces nacres sont en général précisément dénuées d'une bonne aptitude à être compactée avec des liants dits classiques et leur présence à un taux élevé dans une fard à paupières sous forme de poudre compacte peut affecter significativement la cohésion de la poudre résultante, voire lorsqu'elles sont en quantité importante, rendre impossible l'obtention d'une poudre compacte correspondante au moyen d'une presse mécanique.

Il faut alors augmenter le taux de liant pour améliorer le compactage, par exemple à une échelle de 10 % en poids de liant dans la composition, ce qui s'avère généralement préjudiciable à l'aspect de surface qui est alors trop gras.

Il existe donc un besoin pour une composition cosmétique de type poudre compacte comprenant des quantités importantes de pigments nacrés et présentant un effet coloriel intense, tout en étant pourvue de caractéristiques de cohésion et de délitage satisfaisantes, et ne soit pas par ailleurs contraignante en terme de préparation industrielle.

Les inventeurs ont constaté de manière surprenante qu'une telle composition peut être obtenue en utilisant, dans un fard à paupières sous forme de poudre compacte, des agents de coloration particuliers, en particulier des nacres, avec une phase grasse liquide comprenant du polydécène liquide à 25 °C associé à une cire, qui est en particulier sous forme de poudre, et/ou une poudre de polyuréthane.

Cette association permet l'obtention d'une composition apte à former sur les matières kératiniques un film présentant un effet coloriel intense, en outre, le film de composition obtenu est lisse et homogène et présente une brillance intense. La composition présente une bonne stabilité au stockage, ne s'altère pas au cours du temps lorsqu'elle subit des chocs ; elle présente également une bonne aptitude au prélèvement.

De façon plus précise, l'invention a pour objet un fard à paupières sous forme de poudre compacte comprenant, dans un milieu cosmétiquement acceptable, une phase grasse comprenant du polydécène liquide à 25 °C, au moins une cire présente totalement ou partiellement sous forme de poudre et au moins un agent de coloration choisi parmi les pigments diffractants, les pigment interférentiels, les particules réféchissantes et leurs mélanges, ledit fard à paupières présentant une brillance à 45° supérieure ou égale à 1000 coûts.

Selon un second aspect, l'invention a pour objet un fard à paupières sous forme de poudre compacte comprenant, dans un milieu cosmétiquement acceptable, une phase grasse comprenant du polydécène liquide à 25 °C, au moins une poudre de polyuréthane et au moins un agent de coloration choisi parmi les pigments diffractants, les pigments interférentiels, les particulesréféchissantes et leurs mélanges, ledit fard à paupières présentant une brillance à 45° supérieure ou égale à 1000 coûts.

Selon un troisième aspect, l'invention a pour objet un fard à paupières comprenant, dans un milieu cosmétiquement acceptable, une phase grasse comprenant du polydécène liquide à 25 °C et au moins 51% d'au moins un agent de coloration choisi parmi les pigments diffractants, les pigments interférentiels, les particules réféchissantes et leurs mélanges.

L'invention a aussi pour objet un procédé cosmétique de maquillage des paupières, comprenant l'application sur les paupières, du fard à paupières tel que défini précédemment.

L'invention a également pour objet l'utilisation d'un fard à paupières tel que défini précédemment pour l'obtention d'un maquillage, déposé sur les paupières, brillant

### Brillance

La brillance est mesurée selon le protocole suivant :

### a) Préparation de l'échantillon

On prélève le fard à paupières sous forme compactée à l'aide d'un morceau d'éponge du type de celle commercialisée dans le produit « Photogénic » de Lancôme qui a été préalablement découpée à l'aide d'un emporte pièce de 18 mm et collée sur un capuchon de stick de conditionnement de rouge à lèvres à l'aide d'un adhésif double face.
Le stick comprenant l'échantillon est fixé sur un appareil de délitage (Ref. BIEN 010778) paramétré de la façon suivante : Force appliquée (réglée à l'aide de masselotes) : 100g ; mouvement des engrenages : position 7/7 ; nombre de tours : 5. L'échantillon de fard à paupières est étalé via l'appareil sur un morceau de carte de contraste noir mat (Leneta form WP1) recouvert d'une plaquette de Blenderm® (3M Santé) de dimension 40mm x 70mm. Le dépôt de fard à paupières est de 0,5 mg/cm2 et présente une épaisseur de 0,35 mm.

### b) Mesure de la brillance

La brilllance du fard à paupières est évaluée par illumination de l'échantillon, préparé selon le point a), déposé sur la carte de contraste et mesure de l'intensité lumineuse réfléchie par l'échantillon en fonction de l'angle d'observation, à l'aide d'un Spectrogoniophotomètre GON 360 de la société Instrument Systems.
L'angle d'illumination est fixée à 45°, la mesure de l'intensité réfléchie est lue à 45°, avec un temps d'intégration de 1 seconde.
La valeur de la brilllance est exprimée en coûts.

La fard à paupières selon l'invention présente une brillance à 45° supérieure ou égale à 1000 coûts, de préférence supérieure ou égale à 2000 coûts, encore mieux supérieure ou égale à 2500 coûts, pouvant aller jusqu'à 10000 coûts.

### Agent de coloration

L'agent de coloration est choisi parmi les pigments diffractants, les pigments interférentiels, les pigments réféchissants et leurs mélanges. Il peut être présent dans le fard à paupières selon l'invention en une teneur allant de 40 à 90% en poids, de préférence allant de 50 à 80% en poids, et mieux de 55 à 70% en poids par rapport au poids total de la composition, en particulier de 60 à 70% en poids.

En particulier, l'agent de coloration est présent en une teneur supérieure ou égale à 51% en poids, de préférence supérieure ou égale 55% en poids, mieux supérieure ou égale à 60% en poids, et encore mieux supérieure ou égale à 65% en poids par rapport au poids total de la composition.

### PIGMENT INTERFERENTIEL

L'expression « pigment interférentiel » désigne un pigment capable de produire une couleur par un phénomène d'interférences, par exemple entre la lumière réfléchie par une pluralité de couches superposées d'indices de réfraction différents, notamment une succession de couches de haut et de bas indices de réfraction.
Un pigment interférentiel peut par exemple comporter plus de quatre couches d'indices de réfraction différents.
Les couches du pigment interférentiel peuvent entourer ou non un noyau, lequel peut présenter une forme aplatie ou non.
Les nacres sont des exemples de pigments interférentiels.

### Nacres

Par « nacre », il faut comprendre des particules colorées de toute forme, irisées ou non, notamment produites par certains mollusques dans leur coquille ou bien synthétisées et qui présentent un effet de couleur par interférence optique.
Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.
Les nacres peuvent plus particulièrement posséder une couleur ou un reflet jaune, rose, rouge, bronze, orangé, brun, or et/ou cuivré.
A titre illustratif des nacres pouvant être introduites en tant que pigment interférentiel dans la première composition, on peut citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

On utilise de préférence une matière colorante choisie parmi les nacres, en particulier les micas recouverts d'au moins une couche d'oxyde métallique.

### Particules réfléchissantes interférentielles

Ces particules peuvent être choisies parmi les particules à substrat synthétique enrobé au moins partiellement d'au moins une couche d'au moins un oxyde métallique, choisi par exemple parmi les oxydes de titane, notamment TiO₂, de fer notamment Fe₂O₃, d'étain, de chrome, le sulfate de baryum et les matériaux suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.
A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS^{®} par la société ENGELHARD.

### Pigment goniochromatique

Par « pigment goniochromatique », on désigne au sens de la présente invention un pigment permettant d'obtenir, lorsque la composition est étalée sur un support, un trajet de couleur dans le plan a*b* de l'espace colorimétrique CIE 1976 qui correspond à une variation Dh° de l'angle de teinte h° d'au moins 20° lorsque l'on fait varier l'angle d'observation par rapport à la normale entre 0° et 80°, pour un angle d'incidence de la lumière de 45°.
Le trajet de couleur peut être mesuré par exemple au moyen d'un spectrogonioréflectomètre de marque INSTRUMENT SYSTEMS et de référence GON 360 GONIOMETER, après que la première composition a été étalée à l'état fluide avec une épaisseur de 300 µm au moyen d'un étaleur automatique sur une carte de contraste de marque ERICHSEN et de référence Typ 24/5, la mesure étant effectuée sur le fond noir de la carte.
Le pigment goniochromatique peut être choisi par exemple parmi les structures multicouche interférentielles et les agents de coloration à cristaux liquides.
Dans le cas d'une structure multicouche, celle-ci peut comporter par exemple au moins deux couches, chaque couche étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.
La structure multicouche peut présenter ou non, par rapport à une couche centrale, une symétrie au niveau de la nature chimique des couches empilées. Selon l'épaisseur et la nature des différentes couches, on obtient différents effets.
Des exemples de structures multicouche interférentielles symétriques sont par exemple les structures suivantes : Fe₂O₃/SiO₂/Fe₂O₃/SiO₂/Fe₂O₃, un pigment ayant cette structure étant commercialisé sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂, des pigments ayant ces structures étant commercialisés sous la dénomination XIRONA par la société MERCK (Darmstadt).
Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE^{®} HC par la société WACKER.
Comme pigment goniochromatique, on peut encore utiliser certaines nacres, des pigments à effets sur substrat synthétique, notamment substrat type alumine, silice, borosilicate, oxyde de fer, aluminium, ou des paillettes interférentielles issues d'un film de polytéréphthalate.
Le matériau peut en outre comporter des fibres goniochromatiques dispersées. De telles fibres pourront présenter une longueur inférieure à 80 µm par exemple.

### PIGMENT DIFFRACTANT

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière. Un tel pigment est encore parfois appelé pigment holographique.

Un pigment diffractant peut comporter un réseau de diffraction, capable par exemple de diffracter dans des directions définies un rayon de lumière monochromatique incident.
Le réseau de diffraction peut comporter un motif périodique, notamment une ligne, la distance entre deux motifs adjacents étant du même ordre de grandeur que la longueur d'onde de la lumière incidente.
Lorsque la lumière incidente est polychromatique, le réseau de diffraction va séparer les différentes composantes spectrales de la lumière et produire un effet arc-en-ciel.
On pourra utilement se reporter concernant la structure des pigments diffractants à l'article « *Pigments Exhibiting Diffractive Effects* » d'Alberto Argoitia and Matt Witzman, 2002, Society of Vacuum coaters, 45^{th} Annual Technical Conference Proceedings 2002.
Le pigment diffractant peut être réalisé avec des motifs ayant différents profils, notamment triangulaires, symétriques ou non, en créneaux, de largeur constante ou non, sinusoïdaux, en escalier.
La fréquence spatiale du réseau et la profondeur des motifs seront choisies en fonction du degré de séparation des différents ordres souhaités. La fréquence peut varier par exemple entre 500 et 3000 lignes par mm.
De préférence, les particules du pigment diffractant présentent chacune une forme aplatie, et notamment sont en forme de plaquette.
Une même particule de pigment peut comporter deux réseaux de diffraction croisés, perpendiculaires ou non, de même linéature ou non.
Le pigment diffractant peut présenter une structure multicouche comportant une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : MgF₂, SiO₂, Al₂O₃, AlF₃, CeF₃, LaF₃, NdF₃, SmF₂, BaF₂, CaF₂, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr, Fe et leurs matériaux, associations, alliages et leur dopage par des terres rares.
Un tel matériau réfléchissant peut, seul, constituer le pigment diffractant qui sera alors monocouche.
En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : MgF₂, SiO, SiO₂, Al₂O₃, TiO₂, WO, AIN, BN, B₄C, WC, TiC, TiN, N₄Si₃, ZnS, des particules de verre, des carbones de type diamant et leurs associations.
En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de SiO₂, de carbone, d'un oxyde de fer/mica, de mica recouvert de BN, de BC, de graphite, d'oxychlorure de bismuth, et leurs associations.
A la place d'une couche d'un matériau diélectrique, d'autres matériaux améliorant les propriétés mécaniques peuvent convenir. De tels matériaux peuvent comporter du silicone, des silicides métalliques, des matériaux semi-conducteurs formés à partir d'éléments des groupes III, IV et V, des métaux ayant une structure cristalline cubique centrée, des compositions ou matériaux de cermet, des verres semi-conducteurs, et leurs associations variées.
Le pigment diffractant utilisé peut notamment être choisi parmi ceux décrits dans la demande de brevet américain US 2003/0031870 publiée le 13 février 2003.
Un pigment diffractant peut comporter par exemple la structure suivante : MgF₂/Al/MgF₂, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du MgF₂ peut être comprise entre 80 et 95 % du poids total du pigment.
D'autres pigments diffractants sont commercialisés sous les dénominations Metalure^{®} Prismatic par la société ECKART^{®}.
D'autres structures possibles sont Fe/Al/Fe ou AI/Fe/Al.
La dimension du pigment diffractant peut être comprise par exemple entre 5 et 200 µm, mieux entre 5 et 100 µm, par exemple entre 5 et 30 µm.

L'épaisseur des particules de pigment diffractant peut être inférieure ou égale à 3 µm, mieux 2 µm, par exemple de l'ordre de 1 µm.

### Pigments ou particules réfléchissantes

Par « particules réfléchissantes », on désigne des particules dont la taille, la structure, notamment l'épaisseur de la ou des couches qui la constituent et leurs natures physique et chimique, et l'état de surface, leur permettent de réfléchir la lumière incidente. Cette réflexion peut, le cas échéant, posséder une intensité suffisante pour créer à la surface de la composition ou du mélange, lorsque celui-ci est appliqué sur le support à maquiller, des points de surbrillance visibles à l'oeil nu, c'est-à-dire des points plus lumineux qui contrastent avec leur environnement en semblant briller.
Les particules réfléchissantes peuvent être sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.
Ces particules peuvent présenter des formes variées, notamment être en forme de plaquettes ou globulaires, en particulier sphériques.
Les particules réfléchissantes, quelle que soit leur forme, peuvent présenter une structure multicouche ou non et, dans le cas d'une structure multicouche, par exemple au moins une couche d'épaisseur uniforme, notamment d'un matériau réfléchissant.
Lorsque les particules réfléchissantes ne présentent pas de structure multicouche, elles peuvent être composées par exemple d'oxydes métalliques, notamment des oxydes de titane ou de fer obtenus par synthèse.
Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou matériau métallique. Le substrat peut être monomatière, multimatériau, organique et/ou inorganique.
Plus particulièrement, il peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.
Le matériau réfléchissant peut comporter une couche de métal ou d'un matériau métallique.
Des particules réfléchissantes sont décrites notamment dans les documents
JP-A-09188830, JP-A-10158450, JP-A-10158541, JP-A-07258460 et JP-A-05017710.
Toujours à titre d'exemple de particules réfléchissantes comportant un substrat minéral enrobé d'une couche de métal, on peut citer également les particules comportant un substrat de borosilicate enrobé d'argent.
Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

On peut également utiliser des particules comprenant un substrat métallique tel que l'argent, l'aluminium, le fer, le chrome, le nickel, le molybdène, l'or, le cuivre, le zinc, l'étain, le magnésium, l'acier, le bronze, le titane, ledit substrat étant enrobé d'au moins une couche d'au moins un oxyde métallique tels que l'oxyde de titane, l'oxyde d'aluminium, l'oxyde de fer, l'oxyde de cérium, l'oxyde de chrome, les oxydes de silicium et leurs mélanges.
On peut citer à titre d'exemple les poudres d'aluminium, de bronze ou de cuivre enrobées de SiO2 commercialisées sous la dénomination VISIONAIRE par la société ECKART.

### Phase grasse liquide

La phase grasse liquide présente dans la composition selon l'invention comprend du polydécène liquide à 25 °C. Le polydécène peut être notamment du polydécène hydrogéné.

Le polydécène a avantageusement un poids moléculaire moyen en poids allant de 800 à 3000, de préférence allant de 1000 à 2500, et préférentiellement allant de 1200 à 2200. Un tel polydécène permet d'obtenir une composition compactée présentant une bonne résistance aux chocs.

Comme polydécène, on peut utiliser ceux vendus sous les dénominations :
- CERAFLOW E par la société SHAMROCK, de poids moléculaire moyen en poids d'environ 1400
- CERAFLOW HE par la société SHAMROCK, de poids moléculaire moyen en poids d'environ 2000

Le polydécène liquide à 25 °C peut être présent dans la composition selon l'invention en une teneur allant de 1 % à 20 en poids, par rapport au poids total de la composition , de préférence allant de 2 % à 15 % en poids, et préférentiellement allant de 3 % à 10 % en poids.

La phase grasse de la composition selon l'invention peut comprendre une huile additionnelle. L'huile additionnelle peut être choisie parmi les huiles utilisées classiquement comme liant dans les poudres compactes. Parmi les huiles additionnelles utilisables, on peut citer l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; les huiles d'hydrocarbures, telles que les huiles de paraffine, le squalane, la vaseline; les esters gras, tels que le myristate d'isopropyle, le palmitate d'isopropyle, le stéarate de butyle, le stéarate d'isodécyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine, le triethyl hexanoate de glycéryle; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées; les acides gras supérieurs tels que l'acide myristique, l'acide palmitique, l'acide stéarique, l'acide béhénique, l'acide oléique, l'acide linoléique, l'acide linolénique ou l'acide isostéarique; les alcools gras supérieurs tels que le cétanol, l'alcool stéarylique ou l'alcool oléique.

### Cire

Selon une variante particulière de l'invention, la fard à paupières comprend au moins une cire présente totalement ou partiellement sous forme de poudre, notamment micronisée, pour faciliter sa mise en oeuvre dans la préparation de la composition cosmétique.

Par "cire", au sens de la présente invention, on entend un composé gras lipophile, solide à température ambiante (25°C) et pression atmosphérique (760 mm Hg, soit 10⁵ Pa), à changement d'état solide/liquide réversible, ayant en particulier une température de fusion supérieure ou égale à 30°C, notamment supérieure ou égale à 55 °C, et pouvant aller jusqu'à 250 °C, notamment jusqu'à 230 °C, et en particulier jusqu'à 120 °C.

En portant la cire à sa température de fusion, il est possible de la rendre miscible aux huiles et de former un mélange homogène microscopiquement, mais en rétablissant la température du mélange à la température ambiante, on obtient une recristallisation de la cire dans les huiles du mélange.
Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER, avec une montée en température de 5 ou 10 °C par minute.
Les cires, au sens de l'invention, peuvent être celles utilisées généralement dans les domaines cosmétiques ou dermatologiques. Elles peuvent notamment être hydrocarbonées, siliconées et/ou fluorées, comportant éventuellement des fonctions ester ou hydroxyle. Elles peuvent être également d'origine naturelle ou synthétique.
A titre illustratif et non limitatif de ces cires, on peut notamment citer :
- la cire d'abeilles, la cire de lanoline, et les cires d'insectes de Chine ; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40 °C et notamment à plus de 55 °C,
- les cires obtenues par hydrogénation catalytique d'huiles animales ou végétales ayant des chaînes grasses, linéaires ou ramifiées, en C₈-C₃₂, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicones ou les cires fluorées, et
- leurs mélanges

Parmi les cires utilisables sous forme de poudre, on peut notamment citer les microbilles de cire de Carnauba vendues sous la dénomination Microcare 350^{®} par la société Micro Powders et les microbilles de cire de paraffine vendues sous la dénomination Microease 114S^{®} par la société Micro Powders.

La cire présente totalement ou partiellement sous forme de poudre peut être présente en une teneur allant de 0,1 à 45% en poids par rapport au poids total de la composition, de préférence de 0,5 à 20% en poids et mieux de 1 à 10% en poids.

### Phase grasse solide :

La composition selon l'invention comporte, outre la cire sous forme de poudre, au moins une phase grasse comprenant au moins une phase grasse solide, dite encore « liant solide ».
Par « liant solide », on entend au sens de la présente invention une phase grasse dont le point de fusion peut être supérieur ou égal à 30 °C, notamment peut aller de 30 à 250 °C et en particulier de 30 à 230 °C.
Cette phase grasse solide peut comprendre au moins un composé choisi parmi les cires dites additionnelles qui ne sont pas sous forme partielle ou totale de poudre, les savons métalliques et leurs mélanges.

Les cires additionnelles peuvent être chosises parmi les cires décrites plus haut.

A titre de savons métalliques, on peut notamment citer les savons métalliques d'acides gras ayant de 12 à 22 atomes de carbone, et en particulier ceux ayant de 12 à 18 atomes de carbone.
Le métal du savon métallique peut notamment être du zinc ou du magnésium.
L'acide gras peut notamment être choisi parmi l'acide laurique, l'acide myristique, l'acide stéarique, l'acide palmitique.
Comme savon métallique, on peut utiliser le laurate de zinc, le stéarate de magnésium, le myristate de magnésium, le stéarate de zinc, et leurs mélanges.
- les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; ces savons, présents généralement sous la forme de particules ayant des dimensions inférieures à 10 µm ;

Selon une variante particulière de l'invention, la phase grasse solide peut comprendre au moins un savon métallique présent totalement ou partiellement sous forme de poudre.

La composition selon l'invention peut comprendre au moins une phase grasse solide en une teneur variant de 0,5 à 45% en poids, notamment de 1 à 20 % en poids, et en particulier de 1 à 10 % en poids par rapport au poids total de la composition.

### Charges

Le fard à paupières selon l'invention peut comprendre au moins une charge qui peut être organique ou minérale, de forme sphérique ou lamellaire.

La charge incompactable peut être organique ou minérale, de forme sphérique ou lamellaire.

Parmi les charges incompactables de forme sphérique, on peut citer :
- les microsphères de silice, notamment à porosité ouverte ou, de préférence, les microsphères de silice creuses, telles que les "SILICA BEADS SB 700/HA" ou "SILICA BEADS SB 700" de la société MAPRECOS, ; ces microsphères peuvent être imprégnées d'un actif cosmétique ;
- les microsphères microporeuses de polymères, qui ont une structure analogue à celle d'une éponge; elles ont, en général, une surface spécifique d'au moins 0,5 m²/g et, en particulier, d'au moins 1 m²/g, ladite surface spécifique n'ayant pas de limite supérieure autre que celle résultant de la possibilité pratique de réaliser des microsphères de porosité très élevée : la surface spécifique peut, par exemple, atteindre 1 000 m²/g ou même davantage. On peut citer les microsphères de polymères acryliques, telles que celles en copolymère d'acrylate réticulé 'Polytrap 6603 Adsorber' de la société RP SCHERER, et celles de polyméthacrylate de méthyle 'MICROPEARL M 100' de la société SEPPIC;
- la poudre de polyuréthane telle que la poudre de copolymère de diisocyanate d'hexaméthylène et de triméthylol hexyl lactone vendue sous les dénomination PLASTIC POWDER D-400 et T-7 par la société TOSHIKI;
- les microcapsules de polymères qui comportent une seule cavité fermée et forment un réservoir, qui peut contenir un liquide, notamment un actif cosmétique; elles sont préparées par des procédés connus tels que ceux décrits dans le brevet US-A 3 615 972 et EP-A 0 56219 . Elles peuvent être réalisées, par exemple, en polymères ou copolymères d'acides, d'amines ou d'esters monomères à insaturation éthylénique, en polymères urée-formaldéhyde, en polymères ou copolymères de chlorure de vinylidène; à titre d'exemple, on peut citer les microcapsules faites de polymères ou copolymères d'acrylate ou de méthacrylate de méthyle, ou encore de copolymères de chlorure de vinylidène et d'acrylonitrile; parmi ces derniers, on indiquera, notamment, ceux qui contiennent, en poids 20-60% de motifs dérivés de chlorure de vinylidène, 20-60% en poids de motifs dérivés d'acrylonitrile et 0-40% en poids d'autres motifs tels que des motifs dérivés d'un monomère acrylique et/ou styrénique; on peut également utiliser des polymères ou copolymères acryliques réticulés ;
- les poudres sphériques d'organopolysiloxane réticulés élastomères, notamment décrites dans le document JP-A-02-243612, telles que celles vendues sous la dénomination "TREFIL POWDER E-506C" par la société DOW CORNING.

Selon un mode de réalisation particulier, le fard à paupières selon l'invention comprend une poudre de polyuréthane, comme par exemple la poudre de copolymère de diisocyanate d'hexaméthylène et de triméthylol hexyl lactone vendue sous les dénomination PLASTIC POWDER D-400 et T-7 par la société TOSHIKI.

La charge incompactable peut être présente en une teneur allant de 0,1 à 45% en poids, notamment de 0,5% à 20% en poids, et en particulier de 1 à 10% en poids par rapport au poids total de la composition.

Comme charges compactables de forme lamellaire, on peut citer :
- les talcs ou silicates de magnésium hydratés, notamment sous forme de particules de dimensions généralement inférieures à 40 µm;
- les micas ou aluminosilicates de compositions variées et qui se présentent notamment sous forme d'écailles ayant des dimensions de 2 à 200 µm, de préférence 5-70 µm et une épaisseur de 0,1 à 5 µm, de préférence de 0,2-3 µm, ces micas pouvant être d'origine naturelle (par exemple muscovite, margarite, roscoelite, lipidolite, biotite) ou d'origine synthétique ;
- les argiles telles que les séricites, qui appartiennent à la même classe chimique et cristalline que la muscovite ;
- le kaolin ou silicate d'aluminium hydraté, qui se présente notamment sous la forme de particules de formes isotropes ayant des dimensions généralement inférieures à 30µm ;
- les nitrures de bore.

Parmi les charges compactables de type lamellaire organique, on peut citer les poudres de polymères de tétrafluoroéthylène, telles que le 'CERIDUST 9205 F' de la société CLARIANT.

Comme charges compactables de forme sphérique, on peut citer :
- les oxydes de zinc et de titane, généralement utilisés sous la forme de particules ayant des dimensions ne dépassant pas quelques micromètres (ou même inférieures à 1 µm dans le cas de l'oxyde de titane) ;
- le carbonate de calcium précipité, notamment sous forme de particules de dimensions supérieures à 10 µm ;
- le carbonate et l'hydrocarbonate de magnésium ;
- l'hydroxyapatite.
- les poudres de polymères synthétiques non expansés, tels que le polyéthylène, les polyesters (par exemple isophtalate ou téréphtalate de polyéthylène) et les polyamides (par exemple le Nylon), sous la forme de particules ayant des dimensions inférieures à 50 µm ;
- les poudres de polymères synthétiques, réticulés ou non, sphéronisées, comme les poudres de polyamides telles que les poudres de poly-β-alanine ou de Nylon, par exemple la poudre 'Orgasol' de la société ATOCHEM, des poudres d'acide polyacrylique ou polyméthacrylique, des poudres de polystyrène réticulé par le divinylbenzène et des poudres de résine de silicone, et
- des poudres de matériaux organiques d'origine naturelle comme les amidons, notamment de maïs, de blé ou de riz.

Les charges compactables peuvent être présentes en une teneur allant de 0,05 à 40% en poids par rapport au poids total de la composition, de préférence allant de 0,1 à 20% en poids.
La composition selon l'invention peut comprendre, outre l'agent de coloration choisi parmi les pigments diffractants, les pigments interférentiels, les particules réféchissantes, au moins un agent de coloration, dit additionnel, produisant une couleur par absorption d'au moins une partie du spectre visible.

Une couleur produite par absorption de la lumière est encore qualifiée parfois de couleur chimique, par opposition aux couleurs produites par un phénomène d'interférences, y compris de diffraction, encore appelées couleurs physiques. Le phénomène d'absorption d'énergie lumineuse par l'agent de coloration peut reposer sur des transitions électroniques.
L'agent de coloration produisant une couleur par un phénomène d'absorption peut être constitué par un pigment organique ou inorganique ou hybride comportant à la fois de la matière organique et de la matière inorganique.
L'agent de coloration peut être un composé particulaire ou non.
Lorsque l'agent de coloration comporte un colorant, celui-ci peut être choisi parmi les colorants liposolubles et hydrosolubles.
Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.
Les colorants hydrosolubles sont par exemple le jus de betterave et le bleu de méthylène.

Les colorants peuvent par exemple représenter de 0,1 à 20 % du poids de la composition, voire de 0,1 à 6 %, lorsque présents.
L'agent de coloration peut encore être une laque ou un pigment organique choisi parmi les matériaux ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.
Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n°31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.
L'agent de coloration peut être une laque organique supportée par un support organique tel que la colophane ou le benzoate d'aluminium, par exemple.
Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.
Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.
L'agent de coloration peut être un pigment composite, comportant un noyau enrobé au moins partiellement par une écorce.
Les pigments inorganiques ou minéraux sont par exemple :
- les oxydes de fer, de titane, de zirconium, de cérium, de zinc, de fer ou de chrome,
- le bleu ferrique, le violet de manganèse, le bleu, le rose ou le violet d'outremer, l'hydrate de chrome, l'hydroxyde de chrome, l'oxychlorure de bismuth,
- et leurs mélanges.

### Pigments composites

Un pigment composite selon l'invention peut être composé notamment de particules comportant :
- un noyau inorganique,
- au moins un enrobage au moins partiel d'au moins une matière colorante organique.

Au moins un liant peut avantageusement contribuer à la fixation de la matière colorante organique sur le noyau inorganique.
Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.
Un pigment composite peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.
La proportion massique du noyau peut excéder 50 % par rapport au poids total du pigment composite, par exemple aller de 50 % à 70 %, par exemple de 60 % à 70 %.

Le fard à paupières selon l'invention peut comprendre au moins une charge qui peut être organique ou minérale, de forme sphérique ou lamellaire.

Le pigment composite peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184 426.
Une autre méthode de fabrication d'un pigment composite est décrite dans le brevet JP 3286463, qui divulgue un procédé de précipitation en solution.

Selon un mode de réalisation, la matière colorante produisant une couleur par absorption d'au moins une partie du spectre visible est présente dans la composition selon l'invention en une teneur inférieure ou égale à 10% en poids, de préférence inférieure ou égale à 5% en poids.

C'est pourquoi l'invention a aussi pour objet un fard à paupières comprenant, dans un milieu cosmétiquement acceptable, une phase grasse comprenant du polydécène liquide à 25 °C ladite composition comprenant moins de 10% d'agent de coloration produisant une couleur par absorption d'au moins une partie du spectre visible.

La composition selon l'invention peut être une composition anhydre, c'est-à-dire une composition contenant moins de 2 % en poids d'eau, voire moins de 0,5 % d'eau, notamment moins de 0,2% d'eau, l'eau n'étant pas ajoutée lors de la préparation de la composition mais correspondant à l'eau résiduelle apportée par les ingrédients mélangés.

La composition selon l'invention peut comprendre au moins un additif cosmétique choisi dans le groupe formé par les conservateurs, les parfums, les vitamines, les agents hydratants, les agents adoucissants, les filtres solaires, les polymères filmogènes, les séquestrants, les agents alcalinisants ou acidifiants.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition peut être préparée en mélangeant les ingrédients de la phase pulvérulentes (charges et pigments) puis en ajoutant la phase grasse sous agitation, le mélange étant ensuite broyé et/ou mélangé vigoureusement, éventuellement tamisé, puis versé dans une coupelle et compacté, par exemple à l'aide d'une presse.
La composition ainsi obtenue se présente sous forme de poudre compacte.

La présente invention est illustrée plus en détail dans les exemples suivants.

### Exemple 1:

On a préparé le fard à paupières compacté suivant :

| | |
|---|---|
| Mica / oxyde de fer noir'(MICRONA MATTE BLACK de Merck) | 70 g |
| Oxydes de fer noir | 4 g |
| Talc | 0,2 g |
| Triisostérate de glycéryle (DUB TGIS de Stéarinerie Dubois) | 8 g |
| Stéarate de magnésium | 3 g |
| Microbilles de cire de paraffine (Microease^{®} 114S de Micro Powders) | 4 g |
| Poudre de polyuréthane et de silice (PLASTIC POWDER D400 de Toshiki) | 2 g |
| Polydécène hydrogéné (Ceraflow E^{®} de Shamrock) | 8 g |
| Conservateurs | 0,8 g |

La composition compactée a une bonne résistance aux chocs et se délite facilement à l'aide d'un applicateur. Le fard à paupières présente une brillance moyenne (à 45°), mesurée selon le protocole indiqué plus haut, de 1017 +/- 3,8% coûts.

### Exemple 2 :

On a préparé le fard à paupières compacté suivant :

| | |
|---|---|
| Alumine enrobée oxyde de titane (XIRONA SILVER de Merck) | 60 g |
| Talc | 15,2 g |
| Triisostérate de glycéryle (DUB TGIS de Stéarinerie Dubois) | 7,5 g |
| Stéarate de magnésium | 3 g |
| Microbilles de cire de paraffine (Microease^{®} 114S de Micro Powders) | 4 g |
| Poudre de polyuréthane et de silice (PLASTIC POWDER D400 de Toshiki) | 2 g |
| Polydécène hydrogéné (Ceraflow E^{®} de Shamrock) | 7,5 g |
| Conservateurs | 0,8 g |

La composition compactée a une bonne résistance aux chocs et se délite facilement à l'aide d'un applicateur. Le fard à paupières présente une brillance moyenne (à 45°), mesurée selon le protocole indiqué plus haut, de 4267 +/- 9,9% coûts

## Revendications

1. Fard à paupières sous forme de poudre compacte comprenant, dans un milieu cosmétiquement acceptable, une phase grasse comprenant du polydécène liquide à 25 °C, au moins une cire présente totalement ou partiellement sous forme de poudre et au moins un agent de coloration choisi parmi les pigments diffractants, les pigments interférentiels, les particules réféchissantes et leurs mélanges, ledit fard à paupières présentant une brillance à 45° supérieure ou égale à 1000 coûts.

2. Fard à paupières sous forme de poudre compacte comprenant, dans un milieu cosmétiquement acceptable, une phase grasse comprenant du polydécène liquide à 25 °C, au moins une poudre de polyuréthane et au moins un agent de coloration choisi parmi les pigments diffractants, les particules réféchissantes, les pigments interférentiels et leurs mélanges, ledit fard à paupières présentant une brillance à 45° supérieure ou égale à 1000 coûts.

3. Fard à paupières sous forme de poudre compacte comprenant, dans un milieu cosmétiquement acceptable, une phase grasse comprenant du polydécène liquide à 25 °C et au moins 51 % d'au moins un agent de coloration choisi parmi les pigments diffractants, les pigments interférentiels, les pigments réféchissants et leurs mélanges.

4. Fard à paupières sous forme de poudre compacte comprenant, dans un milieu cosmétiquement acceptable, une phase grasse comprenant du polydécène liquide à 25 °C ladite composition comprenant moins de 10% d'agent de coloration produisant une couleur par absorption d'au moins une partie du spectre visible.

5. Fard à paupières selon l'une des revendications 1 à 4, **caractérisé en ce que** le polydécène a un poids moléculaire moyen en poids allant de 800 à 3000, de préférence allant de 1000 à 2500, et préférentiellement allant de 1200 à 2200.

6. Fard à paupières selon l'une des revendications précédentes, **caractérisé en ce que** le polydécène est présent dans la composition selon l'invention en une teneur allant de 1 % à 20 en poids, par rapport au poids total de la composition, de préférence allant de 2 % à 15 % en poids, et préférentiellement allant de 3 % à 10 % en poids.

7. Fard à paupières selon l'une des revendications précédentes, **caractérisé en ce que** les pigments diffractants sont choisis parmi :
- les pigments monocouche comprenant un matériau réfléchissant choisi parmi les métaux et leurs alliages,
- les pigments présentant une structure multicouche comportant une couche d'un matériau réfléchissant choisi parmi les métaux et leurs alliages et aussi parmi les matériaux réfléchissants non métalliques, recouverte au moins d'un côté d'une couche d'un matériau diélectrique.
- les pigments composés d'un matériau diélectrique ou céramique préformé tel qu'un minéral en lamelle naturelle, ou des lamelles synthétiques,
et leurs mélanges.

8. Fard à paupières selon l'une des revendications précédentes, **caractérisé en ce que** les pigments interférentiels sont choisis parmi les nacres, les particules réfléchissantes interférentielles, les pigments goniochromatiques et leurs mélanges.

9. Fard à paupières selon la revendication précédente, **caractérisé en ce que** les pigments goniochromatiques sont choisis parmi les structures multicouche interférentielles et les agents de coloration à cristaux liquides.

10. Fard à paupières selon la revendication 8, **caractérisé en ce que** les nacres sont choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique, les pigments nacrés à base d'oxychlorure de bismuth, les particules de mica à la surface desquelles sont superposées au moins deux couches successives d'oxydes métalliques et/ou de matières colorantes organiques et leurs mélanges.

11. Fard à paupières selon la revendication 8, **caractérisé en ce que** les particules réfléchissantes interférentielles sont choisies parmi les particules à substrat synthétique enrobé au moins partiellement d'au moins une couche d'au moins un oxyde métallique.

12. Fard à paupières selon l'une des revendications précédentes, **caractérisé en ce que** les pigments réflechissantes sont choisies parmi
- les oxydes métalliques, notamment les oxydes de titane ou de fer obtenus par synthèse,
- les structures multicouches comprenant un substrat naturel ou synthétique, au moins partiellement enrobé par au moins une couche d'un matériau réfléchissant notamment d'au moins un métal ou matériau métallique.

13. Fard à paupières selon l'une quelconque des revendications 1 ou 2 et 4 à 12, **caractérisé en ce que** l'agent de coloration est présent en une teneur allant de 40 à 90% en poids, de préférence allant de 50 à 80% en poids, et mieux de 55 à 70% en poids par rapport au poids total de la composition à % en poids, par rapport au poids total de la composition, de préférence de 60 à 70% en poids.

14. Fard à paupières selon l'une quelconque des revendications 1 à 12, **caractérisé par le fait que** l'agent de coloration est présent en une teneur supérieure ou égale à 55% en poids, de préférence supérieure ou égale à 60% en poids par rapport au poids total de la composition.

15. Fard à paupières selon l'une quelconque des revendications 2 à 14, **caractérisé en ce qu'**il comprend au moins une cire.

16. Fard à paupières selon l'une quelconque des revendications 2 à 14, **caractérisé en ce que** la cire est présente totalement ou partiellement sous forme de poudre.

17. Fard à paupières selon la revendication 1 et 15, **caractérisé par le fait que** la cire est une cire de paraffine et/ou de carnauba.

18. Fard à paupières selon l'une des revendications 1 et 15 à 17 **caractérisé par le fait que** la cire est présente en une teneur en une teneur allant de 0,1 à 45% en poids par rapport au poids total de la composition, de préférence de 0,5 à 20% en poids et mieux de 1 à 10% en poids.

19. Fard à paupières selon l'une quelconque des revendications 1 et 3 à 13, **caractérisé en ce qu'**il comprend un poudre de polyuréthane.

20. Fard à paupières selon la revendications 1 ou 19, **caractérisé par le fait que** la poudre de polyuréthane est une poudre de copolymère de diisocyanate d'hexaméthylène et de triméthylol hexyl lactone.

21. Fard à paupières selon la revendications 2 ou 19, **caractérisé par le fait que** la poudre de polyuréthane est présente en une teneur allant de 0,1 à 45% en poids, notamment de 0,5% à 20% en poids, et en particulier de 1 à 10% en poids par rapport au poids total de la composition.

22. Fard à paupières selon l'une des revendications précédentes, **caractérisé par le fait qu'**il comprend une phase grasse solide ou liant sec.

23. Fard à paupières selon la revendication précédente, **caractérisé par le fait que** la phase grasse solide comprend au moins un composé choisi parmi les cires, les savons métalliques et leurs mélanges.

24. Fard à paupières selon la revendication 21 ou 23, **caractérisé par le fait que** la phase grasse solide représente de 0,5 à 45% en poids, notamment de 1 à 20 % en poids, et en particulier de 1 à 10 % en poids par rapport au poids total de la composition.

25. Fard à paupières selon l'une des revendications précédentes, **caractérisé par le fait qu'**il comprend au moins une charge compactable.

26. Fard à paupières selon la revendication 25, **caractérisé par le fait que** la charge compactable est présente en une teneur allant de 0,05 à 40% en poids par rapport au poids total de la composition, de préférence allant de 0,1 à 20% en poids.

27. Fard à paupières selon l'une des revendications précédentes, **caractérisé par le fait qu'**il comprend au moins une charge incompactable.

28. Fard à paupières selon la revendication 27, **caractérisé par le fait que** la charge incompactable est présente en une teneur allant de 1 à 45% en poids, notamment de 1% à 20% en poids, et en particulier de 1 à 10% en poids par rapport au poids total de la composition.

29. Procédé de maquillage des paupières comprenant l'application sur les paupières d'un fard à paupières selon l'une quelconque des revendications 1 à 28.

30. Utilisation d'un fard à paupière selon l'une quelconque des revendications 1 à 28 pour l'obtention d'un maquillage, déposé sur les paupières, brillant, homogène et/ou présentant une bonne tenue.
